# EUROPÄISCHE PATENTSCHRIFT

(11) **EP 1 322 578 B1**
(45) Veröffentlichungstag und Bekanntmachung des Hinweises auf die Patenterteilung: **08.03.2006**
(21) Anmeldenummer: 01982292.3
(22) Anmeldetag: 14.09.2001
(51) Int. Cl.: C07C 5/333, C07C 5/48, B01J 21/10, B01J 23/58, B01J 23/62, B01J 23/63

(54) **VERFAHREN ZUR DEHYDRIERUNG VON KOHLENWASSERSTOFFEN**
METHOD FOR THE DEHYDROGENATION OF HYDROCARBONS
PROCEDE POUR DESHYDROGENER DES HYDROCARBURES

(30) Priorität: 26.09.2000 DE 10047642
(43) Veröffentlichungstag der Anmeldung: 02.07.2003
(73) Patentinhaber: BASF Aktiengesellschaft, 67056 Ludwigshafen (DE)
(72) Erfinder: SCHINDLER, Goetz-Peter, 68219 Mannheim (DE); MACHHAMMER, Otto, 68163 Mannheim (DE); HARTH, Klaus, 67317 Altleiningen (DE); MÜLLER-ENGEL, Klaus, Joachim, 76297 Stutensee (DE); ZEHNER, Peter, 67071 Ludwigshafen (DE)
(74) Vertreter: Isenbruck, Günter
(86) Internationale Anmeldenummer: PCT/EP2001/010673
(87) Internationale Veröffentlichungsnummer: WO 2002/026668

(56) Entgegenhaltungen:
- EP-A- 1 074 299
- US-A- 4 886 928
- US-A- 5 633 421
- US-A- 5 733 518

## Beschreibung

Die Erfindung betrifft ein Verfahren zur heterogen katalysierten Dehydrierung von dehydrierbaren C₂-C₃₀-Kohlenwasserstoffen.

Dehydrierte Kohlenwasserstoffe werden als Ausgangsstoffe für zahlreiche industrielle Verfahren in großen Mengen benötigt. Beispielsweise finden dehydrierte Kohlenwasserstoffe bei der Herstellung von Detergentien, klopffestem Benzin und pharmazeutischen Produkten Verwendung. Ebenso werden zahlreiche Kunststoffe durch Polymerisation von Olefinen hergestellt.

Beispielsweise werden aus Propylen Acrylnitril, Acrylsäure oder C₄-Oxoalkohole hergestellt. Propylen wird derzeit überwiegend durch Steamcracken oder durch katalytisches Cracken geeigneter Kohlenwasserstoffe oder Kohlenwasserstoffgemische wie Naphtha hergestellt

Propylen kann darüber hinaus durch heterogen katalysierte Dehydrierung von Propan hergestellt werden.

Um bei heterogen katalysierten Dehydrierungen akzeptable Umsätze auch schon bei einmaligem Reaktor-Durchgang zu erreichen, muss in der Regel bei relativ hohen Reaktionstemperaturen gearbeitet werden. Typische Reaktionstemperaturen für Gasphasendehydrierungen liegen bei 300 bis 700°C. Pro Molekül Kohlenwasserstoff wird dabei in der Regel ein Molekül Wasserstoff erzeugt.

Die Dehydrierung von Kohlenwasserstoffen verläuft endotherm. Die für die Einstellung eines gewünschten Umsatzes benötigte Dehydrierwärme muss entweder dem Reaktionsgas vorab und/oder im Verlauf der katalytischen Dehydrierung zugeführt werden. Bei den meisten bekannten Dehydrierverfahren wird die Dehydrierwärme außerhalb des Reaktors erzeugt und dem Reaktionsgas von außen zugeführt. Dies erfordert jedoch aufwendige Reaktor- und Verfahrenskonzepte und führt insbesondere bei hohen Umsätzen zu steilen Temperaturgradienten im Reaktor, mit der Gefahr einer verstärkten Nebenproduktbildung. So können beispielsweise mehrere adiabate Katalysatorbetten in nacheinander geschalteten Ringspaltreaktoren angeordnet werden. Das Reaktionsgasgemisch wird auf seinem Weg von einem Katalysatorbett zum nächsten Katalysatorbett durch Wärmeaustauscher überhitzt und kühlt sich beim nachfolgenden Reaktordurchgang wieder ab. Um mit einem solchen Reaktorkonzept zu hohen Umsätzen zu gelangen muss entweder die Anzahl der hintereinander geschalteten Reaktoren erhöht werden, oder es muss die Reaktoreingangstemperatur des Gasgemischs erhöht werden. Die dadurch bedingte Überhitzung führt zwangsläufig zu einer verstärkten Nebenproduktbildung durch Crackreaktionen. Ferner ist bekannt, das Katalysatorbett in einem Rohrreaktor anzuordnen und die Dehydrierwärme durch das Verfeuern von brennbaren Gasen außerhalb des Rohrreaktors zu erzeugen und über die Rohrwand in das Innere des Reaktors einzukoppeln. Bei diesen Reaktoren führen hohe Umsätze zu steilen Temperaturgradienten zwischen der Wand und dem Inneren des Reaktionsrohres.

Eine Alternative ist die Erzeugung der Dehydrierwärme direkt in dem Reaktionsgasgemisch der Dehydrierung durch Oxidation von bei der Dehydrierung gebildetem oder zusätzlich zugeführtem Wasserstoff oder von im Reaktionsgasgemisch enthaltenen Kohlenwasserstoffe mit Sauerstoff. Dazu wird dem Reaktionsgasgemisch entweder bereits vor dem ersten Katalysatorbett oder vor nachfolgenden Katalysatorbetten ein sauerstoffhaltiges Gas und gegebenenfalls Wasserstoff zugesetzt. Die bei der Oxidation freigesetzte Reaktionswärme verhindert auch bei hohen Umsätzen große Temperaturgradienten im Reaktor. Gleichzeitig wird durch den Verzicht auf die indirekte Reaktorheizung ein sehr einfaches Verfahrenskonzept verwirklicht.

US 4,788,371 beschreibt ein Verfahren zur Wasserdampfdehydrierung von dehydrierbaren Kohlenwasserstoffen in der Gasphase in Verbindung mit einem oxidativen Wiedererhitzen der Zwischenprodukte, wobei der gleiche Katalysator für die selektive Oxidation von Wasserstoff und die Wasserdampfdehydrierung eingesetzt wird. Dabei kann Wasserstoff als Co-Feed zugeführt werden. Der verwendete Katalysator enthält ein Edelmetall der Gruppe VIII, ein Alkalimetall und ein weiteres Metall aus der Gruppe B, Ga, In, Ge, Sn, und Pb auf einem anorganischen Oxidträger wie Aluminiumoxid. Das Verfahren kann ein-oder mehrstufig in einem Fest- oder Wanderbett durchgeführt werden.

US 4, 886,928 offenbart ein Verfahren zur Dehydrierung von C₂-C₁₅ Kohlenwasserstoffen an einem Katalysator, welcher auf einem anorganischen Oxidtrager ein Platingruppenmetall, ein Element der III. Nebengruppe, ein Element der IV. Hanptgruppe und gegebenenfalls ein Alkali-oder Erdalkalimetall enthält, wobei das Verfahren in Gegenwart eines Sauerstoffhattigen Co. Feed durch geführt werden kann.

WO 94/29021 beschreibt einen Katalysator, der einen im wesentlichen aus einem gemischten Oxid von Magnesium und Aluminium Mg(Al)O bestehenden Träger enthält sowie ein Edelmetall der Gruppe VIII, vorzugsweise Platin, ein Metall der Gruppe IVA, vorzugsweise Zinn, und gegebenenfalls ein Alkalimetall, vorzugsweise Cäsium. Der Katalysator wird in der Dehydrierung von Kohlenwasserstoffen eingesetzt, wobei in Gegenwart von Sauerstoff gearbeitet werden kann.

US 5,733,518 beschreibt ein Verfahren zur selektiven Oxidation von Wasserstoff mit Sauerstoff in Gegenwart von Kohlenwasserstoffen wie n-Butan an einem Katalysator enthaltend ein Phosphat von Germanium, Zinn, Blei, Arsen, Antimon oder Bismuth, vorzugsweise Zinn. Durch die Verbrennung des Wasserstoffs wird die für die endotherme Dehydrierung notwendige Reaktionswärme in wenigstens einer Reaktionszone erzeugt.

EP-A 0 838 534 beschreibt einen Katalysator für die wasserdampffreie Dehydrierung von Alkanen, insbesondere von Isobutan, in Gegenwart von Sauerstoff. Der verwendete Katalysator umfasst ein Platingruppenmetall, welches auf einem Träger aufgebracht ist aus Zinnoxid/Zirkoniumoxid mit mindestens 10% Zinn. Der Sauerstoffgehalt im Einsatzstrom der Dehydrierung wird so abgestimmt, dass die durch Verbrennung von Wasserstoff mit Sauerstoff erzeugte Wärmemenge gleich der für die Dehydrierung benötigten Wärmemenge ist.

WO 96/33151 beschreibt ein Verfahren zur Dehydrierung eines C₂-C₅-Alkans in Abwesenheit von Sauerstoff an einem Dehydrierkatalysator, enthaltend Cr, Mo, Ga, Zn oder ein Gruppe VIII-Metall unter gleichzeitiger Oxidation von gebildetem Wasserstoff an einem reduzierbaren Metalloxid wie den Oxiden von Bi, In, Sb, Zn, Tl, Pb oder Te. Die Dehydrierung muss dabei regelmäßig unterbrochen werden, um das reduzierte Oxid wieder mit einer Sauerstoffquelle zu reoxidieren. In US 5,430,209 ist ein entsprechendes Verfahren beschrieben, bei dem der Dehydrierungsschritt und der Oxidationsschritt nacheinander ablaufen und die dazugehörigen Katalysatoren räumlich voneinander getrennt sind. Als Katalysatoren für die selektive Wasserstoff-Oxidation kommen Oxide des Bi, Sb und Te sowie deren Mischoxide zum Einsatz.

WO 96/33150 beschreibt schließlich ein Verfahren, in dem in einer ersten Stufe ein C₂-C₅₋Alkan an einem Dehydrierungskatalysator dehydriert wird, das Austrittsgas der Dehydrierungsstufe mit Sauerstoff gemischt wird und in einer zweiten Stufe über einen Oxidationskatalysator, bevorzugt Bi₂O₃, geleitet wird, wobei der gebildete Wasserstoff selektiv zu Wasser oxidiert wird, und in einer dritten Stufe das Ausgangsgas der zweiten Stufe erneut über einen Dehydrierungskatalysator geleitet wird.

Das eingesetzte Katalysatorsystem muss hinsichtlich erzielbarem Alkan-Umsatz, Selektivität für die Bildung von Alkenen, mechanischer Stabilität, thermischer Stabilität, Verkokungsverhalten, Deaktivierungsverhalten, Regenerierbarkeit, Stabilität in Gegenwart von Sauerstoff und Unempfindlichkeit gegenüber Katalysatorgiften wie CO, schwefel- und chlorhaltigen Verbindungen, Alkinen usw. und Wirtschaftlichkeit hohen Anforderungen genügen.

Die Katalysatoren aus dem Stand der Technik genügen diesen Anforderungen, insbesondere hinsichtlich der erzielbaren Umsätze und Selektivitäten, Standzeiten und Regenerierbarkeit nicht in ausreichendem Maße.

Aufgabe der Erfindung ist, ein Verfahren zur Dehydrierung von Kohlenwasserstoffen bereitzustellen, das hohe Umsätze, Raum-Zeit-Ausbeuten und Selektivitäten gewährleistet.

Gelöst wird die Aufgabe durch ein Verfahren zur heterogen katalysierten Dehydrierung in einer oder mehreren Reaktionszonen von einem oder mehreren dehydrierbaren C₂-C₃₀₋Kohlenwasserstoffen in einem diese enthaltenden Reaktionsgasgemisch, wobei zumindest ein Teil der benötigten Dehydrierwärme in mindestens einer Reaktionszone durch Verbrennung von Wasserstoff, des oder der Kohlenwasserstoffe und/oder von Kohlenstoff in Gegenwart eines sauerstoffhaltigen Gases direkt in dem Reaktionsgasgemisch erzeugt wird, dadurch gekennzeichnet, dass das Reaktionsgasgemisch, welches den oder die dehydrierbaren Kohlenwasserstoffe enthält, mit einem Lewis-aciden Dehydrierungskatalysator in Kontakt gebracht wird, der im wesentlichen keine Brönsted-Acidität aufweist, wobei der Dehydrierungskatalysator eine Lewis-Acidität größer 3 Aciditätseinheiten, bestimmbar aus IR-Absorptionsspektren von an dem Katalysator absorbiertem Pyridin, aufweist.

Der erfindungsgemäß eingesetzte Dehydrierungskatalysator weist im wesentlichen keine Brönsted-Acidität, aber eine hohe Lewis-Acidität auf. Die Bestimmung der Lewis- und Brönsted-Acidität der Dehydrierungskatalysatoren erfolgt durch Adsorption von Pyridin als basischem Sondenmolekül am aktivierten Katalysator mit anschließender FT-IRspektrometrischer quantitativer Erfassung der Brönsted- bzw. Lewis-spezifischen Adsorbate. Dabei wird die Tatsache ausgenutzt, dass die adsorbierten Sondenmoleküle unterschiedliche IR-Spektren ergeben, je nach dem, ob sie an ein Brönsted- oder an ein Lewis-Zentrum gebunden sind. Am Brönsted-Zentrum findet ein Protonentransfer statt, und es wird ein lokales Ionenpaar mit dem Pyridinium-Ion als Kation gebildet. Das adsorbierte Pyridinium-Ion zeigt im IR-Spektrum eine Brönsted-spezifische Absorptionsbande bei 1545 cm⁻¹. Am Lewis-Zentrum hingegen findet eine koordinative Bindung des Sondenmoleküls Pyridin über sein freies Elektronenpaar am Ring-Stickstoff mit einer Elektronendefizienz statt. Hieraus resultiert ein zum Brönsted-Adsorbat unterschiedliches IR-Spektrum. Die Lewis-Bande ist bei 1440 cm⁻¹ zu finden. Durch quantitative Auswertung der Brönsted- bzw. Lewis-Banden können die Brönsted- bzw.

Lewis-Zentren separat erfasst werden. Die Bandenzuordnung beruht auf der Arbeit von Turkevich (C.H. Kline, J. Turkevich: J. Chem. Phys. 12, 300 (1994)).

Die Zuordnung der sich ergebenden Pyridinbanden im FT-IR-Spektrum ist wie folgt:

| | |
|---|---|
| Lewis (L) : | 1440 cm⁻¹ |
| Brönstedt (B) : | 1545 cm⁻¹ |
| Kontrollbande B+L: | 1490 cm⁻¹ |
| Physisorbiertes Pyridin: | 1590 cm⁻¹ (zusätzlich 1440cm⁻¹) |

Bei der verwendeten Transmissions-Messzelle handelt es sich um einen Nachbau des Prototyps von Gallei und Schadow (E. Gallei et al.: Rev. Sci. Instrum. 45 (12), 1504 (1976)). Die Zelle besteht aus einem Edelstahl-Korpus mit parallel angeordneten IRdurchlässigen Fenstern aus CaF₂. Durch die Eigenabsorption der Fenster ist nur ein Spektralbereich von ca. 1200 - 4000 cm⁻¹ zugänglich. Im Zellkorpus befindet sich ein Kreislauf für Kühl- bzw. Heizflüssigkeit. Im Zelldeckel befindet sich ein massiver, plattenförmiger Probenhalter mit eingebauter Patronenheizung (400 °C). In eine ringförmige Doppelschablone wird der freitragende Probenpressling eingelegt und in die Heizplatte eingeschraubt und der Zelldeckel mit dem Zellkorpus verschraubt. Die Messzelle ist auf 10⁻⁵ bis 10⁻⁶ mbar evakuierbar.

Zur Probenvorbereitung wird das Katalysatormaterial in einem Mörser fein gemahlen und zwischen zwei Edelstahlplatten mit Unterlagen aus Glimmer in einer Filmpresse mit 50 kN Pressdruck zu einem freitragenden Wafer verpresst. Die Schichtdicke richtet sich nach der IR-Eigenabsorption des Materials und liegt typischerweise zwischen 30 und 100 µm. Aus dem Wafer werden Pellets mit einem Durchmesser von ca. 5 mm ausgeschnitten.

Die Aktivierung der Probe in der Messzelle geschieht unter Luft bei einer Temperatur von 390°C. Nach dem Aufheizen wird auf 10⁻⁵ bis 10⁻⁶ mbar evakuiert. Danach wird unter Hochvakuum auf die Begasungstemperatur von 80°C abgekühlt.

Anschließend wird bei einem Begasungsdruck, der zwischen 10⁻² und 3 mbar liegen kann, mit Pyridin begast. Es werden so lange Kontrollspektren der Probe mit Adsorbat aufgenommen, bis sich ein stationärer Adsorptionszustand bei dem betreffenden Begasungsdruck eingestellt hat. Anschließend wird auf Hochvakuum (10⁻⁵ mbar) evakuiert. Dabei werden Physisorbate entfernt. Nach erfolgter Evakuierung werden Adsorbatspektren aufgenommen.

Zur Bestimmung der Lewis- und Brönsted-Acidität werden die sich für eine bestimmte Schichtdicke der Probe und für einen eingestellten Gleichgewichtsdruck des Pyridins ergebenden Intensitäten der Banden bei 1440 cm⁻¹ und 1545 cm⁻¹ vergleichend ausgewertet. Falls keine Bande bei 1545 cm⁻¹ erkennbar ist (keine Brönsted-Acidität) kann auch die Bande bei 1490 cm⁻¹ für die Bestimmung der Lewis-Acidität ausgewertet werden.

Die gemessenen Extinktionen werden auf die Schichtdicke der Probe bezogen (in integralen Extinktionseinheiten (IEE) pro µm Schichtdicke). Als Untergrund der Adsorbatspektren dient das Single-beam-Spektrum der unbegasten Probe (auf 80°C abgekühlt) unter Hochvakuum. Matrixbanden werden dabei vollständig wegkompensiert. 1 AU entspricht dem tausendfachen der gemessenen Extinktion (angegegeben in integralen Extinktionseinheiten IEE) geteilt durch die Schichtdicke der Probe (in µm), die sich bei der Bestimmung der Lewis- und Brönsted-Acidität der Dehydrierungskatalysatoren mit dem Sondengas Pyridin ergibt.

Die erfindungsgemäß eingesetzten Dehydrierungskatalysatoren weisen keine nachweisbare Brönsted-Acidität auf, das heißt ihre Brönsted-Acidität ist kleiner als 0,1 AU. Sie weisen aber eine hohe Lewis-Acidität auf. Die Lewis-Acidität der Dehydrierungskatalysatoren ist größer 3 AU, besonders bevorzugt größer 6 AU.

Die erfindungsgemäß eingesetzten Dehydrierungskatalysatoren weisen im allgemeinen einen Träger und eine Aktivmasse auf. Der Träger besteht dabei aus einem wärmebeständigen Oxid oder Mischoxid. Bevorzugt enthält der Dehydrierungskatalysator ein Metalloxid, das ausgewählt ist aus der Gruppe bestehend aus Zirkondioxid, Zinkoxid, Aluminiumoxid, Siliciumdioxid, Titandioxid, Magnesiumoxid, Lanthanoxid, Ceroxid und deren Gemischen, als Träger. Bevorzugte Träger sind Zirkondioxid und/oder Siliziumdioxid, besonders bevorzugt sind Gemische aus Zirkondioxid und Siliziumdioxid.

Die Aktivmasse des erfindungsgemäß eingesetzten Dehydrierungskatalysators enthält im allgemeinen ein oder mehrere Elemente der VIII. Nebengruppe, bevorzugt Platin und/oder Palladium, besonders bevorzugt Platin. Darüber hinaus kann der Dehydrierungskatalysator ein oder mehrere Elemente der I. und/oder II. Hauptgruppe aufweisen, bevorzugt Kalium und/oder Cäsium. Weiterhin kann der Dehydrierungskatalysator ein oder mehrere Elemente der III. Nebengruppe einschließlich der Lanthaniden und Actiniden enthalten, bevorzugt Lanthan und/oder Cer. Schließlich kann der Dehydrierungskatalysator ein oder mehrere Elemente der III. und/oder IV. Hauptgruppe aufweisen, bevorzugt ein oder mehrere Elemente aus der Gruppe bestehend aus Bor, Gallium, Silizium, Germanium, Zinn und Blei, besonders bevorzugt Zinn.

In einer bevorzugten Ausführungsform enthält der Dehydrierungskatalysator mindestens ein Element der VIII. Nebengruppe, mindestens ein Element der I. und/oder II.

Hauptgruppe, mindestens ein Element der III. und/oder IV. Hauptgruppe und mindestens ein Element der III. Nebengruppe einschließlich der Lanthaniden und Actiniden.

Zur Herstellung der erfindungsgemäß eingesetzten .Dehydrierungskatalysatoren können Precursoren für Oxide des Zirkons, Siliciums, Aluminiums, Titans, Magnesiums, Lanthans oder Cers, die sich durch Calcinieren in die Oxide umwandeln lassen, eingesetzt werden. Diese können nach bekannten Verfahren, zum Beispiel nach dem Sol-Gel-Verfahren, Fällung der Salze, Entwässern der entsprechenden Säuren, Trockenmischen, Aufschlämmen oder Sprühtrocknen hergestellt werden. Zum Beispiel kann zur Herstellung eines ZrO₂•Al₂O₃•SiO₂-Mischoxides zunächst ein wasserreiches Zirkonoxid der allgemeinen Formel ZrO₂•xH₂O durch Fällung eines geeigneten Zirkon enthaltenden Precursors hergestellt werden. Geeignete Precursoren des Zirkons sind zum Beispiel Zr(NO₃)₄, ZrOCl₂, oder ZrCl₄. Die Fällung selbst erfolgt durch Zugabe einer Base wie zum Beispiel NaOH, KOH, Na₂CO₃ und NH₃ und ist beispielsweise in der EP-A 0 849 224 beschrieben.

Zur Herstellung eines ZrO₂•SiO₂-Mischoxides kann der zuvor erhaltene Zirkon enthaltende Precursor mit einem Silizium enthaltenden Precursor gemischt werden. Gut geeignete Precursoren für SiO₂ sind zum Beispiel wasserhaltige Sole des SiO₂ wie Ludox™. Die Mischung der beiden Komponenten kann beispielsweise durch einfaches mechanisches Vermischen oder durch Sprühtrocknen in einem Sprühturm erfolgen.

Zur Herstellung eines ZrO₂•SiO₂•Al₂O₃-Mischoxides kann die wie oben beschrieben erhaltene SiO₂•ZrO₂-Pulvermischung mit einem Aluminium enthaltenden Precursor versetzt werden. Dies kann zum Beispiel durch einfaches mechanisches Mischen in einem Kneter erfolgen. Die Herstellung ZrO₂•SiO₂•Al₂O₃-Mischoxides kann aber auch in einem einzigen Schritt durch Trockenmischung der einzelnen Precursoren erfolgen.

Die Träger für die erfindungsgemäß eingesetzten Dehydrierungskatalysatoren haben unter anderem den Vorteil, dass sie sich leicht verformen lassen. Dazu wird die erhaltene Pulvermischung im Kneter mit einer konzentrierten Säure versetzt und dann in einen Formkörper, z.B. mittels einer Strangpresse oder eines Extruders, überfuhrt.

Die erfindungsgemäß eingesetzten Dehydrierungskatalysatoren besitzen in besonderen Ausgestaltungsformen eine definierte Porenstruktur. Bei der Verwendung von Mischoxiden besteht die Möglichkeit der gezielten Beeinflussung der Porenstruktur. Die Korngröße der verschiedenen Precursoren beeinflussen das Porengefüge. So lassen sich beispielsweise über die Verwendung von Al₂O₃ mit einem geringen Glühverlust und einer definierten Korngrößenzusammensetzung Makroporen im Gefüge erzeugen. Bewährt hat sich in diesem Zusammenhang die Verwendung von Al₂O₃ mit einem Glühverlust von etwa 3% (z. B. Puralox®).

Eine weitere Möglichkeit zur gezielten Herstellung der Träger mit speziellen Porenradienverteilungen für die erfindungsgemäß eingesetzten Dehydrierungskatalysatoren besteht in der Zugabe verschiedener Polymere während der Herstellung, die durch Calcinierung teilweise oder vollständig entfernt werden, wobei Poren in definierten Porenradienbereichen entstehen. Die Mischung der Polymere und der Oxid-Precursoren kann beispielsweise durch einfaches mechanisches Vermischen oder durch Sprühtrocknen in einem Sprühturm erfolgen.

Besonders bewährt zur Herstellung der Träger mit bimodaler Porenradienverteilung hat sich die Verwendung von PVP (Polyvinylpyrrolidon). Wird dieses in einem Herstellschritt zu einem oder mehreren Oxid-Precursoren für Oxide der Elemente Zr, Ti, Al oder Si gegeben, so entstehen nach dem Calcinieren Makroporen im Bereich von 200 bis 5000 nm. Ein weiterer Vorteil der Verwendung von PVP ist die leichtere Verformbarkeit des Trägers. So können aus frisch gefälltem wasserhaltigem ZrO₂•x H₂O, das vorher bei 120°C getrocknet wurde unter Zusatz von PVP und Ameisensäure auch ohne weitere Oxid-Precursoren mühelos Stränge mit guten mechanischen Eigenschaften hergestellt werden.

Die Calcinierung der Träger für die erfindungsgemäß eingesetzten Dehydrierungskatalysatoren erfolgt zweckmäßigerweise nach dem Aufbringen der Aktivkomponenten und wird bei Temperaturen von 400 bis 1000°C, bevorzugt von 500 bis 700°C, besonders bevorzugt bei 550 bis 650°C und insbesondere bei 560 bis 620°C durchgeführt. Die Calcinierungstemperatur sollte dabei üblicherweise mindestens so hoch sein wie die Reaktionstemperatur der Dehydrierung, bei welcher die erfindungsgemäßen Dehydrierungskatalysatoren eingesetzt werden.

Die Träger der erfindungsgemäß eingesetzten Dehydrierungskatalysatoren weisen nach der Calcinierung im allgemeinen hohe BET-Oberflächen auf. Die BET-Oberflächen sind im allgemeinen größer als 40 m²/g, bevorzugt größer als 50 m²/g, besonders bevorzugt größer als 70 m²/g. Das Porenvolumen der erfindungsgemäßen Dehydrierungskatalysatoren beträgt üblicherweise 0,2 bis 0,6 ml/g, bevorzugt 0,25 bis 0,5 ml/g. Der durch Hg-Porosimetrie bestimmbare mittlere Porendurchmesser der erfindungsgemäßen Dehydrierungskatalysatoren liegt zwischen 3 und 20 nm, bevorzugt zwischen 4 und 15 nm.

Charakteristisch für die erfindungsgemäß eingesetzten Dehydrierungskatalysatoren ist weiterhin eine bimodale Porenradienverteilung. Die Poren liegen dabei im Bereich bis 20 nm und zwischen 40 und 5000 nm. Bezogen auf das gesamte Porenvolumen des Dehydrierungskatalysators besitzen diese Poren in Summe mindestens einen Anteil von 70%. Der Anteil an Poren kleiner als 20 nm beträgt dabei im allgemeinen zwischen 20 und 60%, der Anteil an Poren zwischen 40 und 5000 nm beträgt im allgemeinen ebenfalls 20 bis 60%.

Die Aufbringung der dehydrieraktiven Komponente, die üblicherweise ein Metall der VIII. Nebengruppe ist, erfolgt in der Regel durch Tränkung mit einem geeigneten Metallsalzprecursor. Statt durch Tränkung kann die dehydrieraktive Komponente aber auch durch andere Verfahren wie beispielsweise Aufsprühen des Metallsalzprecursors erfolgen. Geeignete Metallsalzprecursoren sind z.B. die Nitrate, Acetate und Chloride der entsprechenden Metalle, möglich sind auch komplexe Anionen der verwendeten Metalle. Bevorzugt wird Platin als H₂PtCl₆ oder Pt(NO₃)₂ eingesetzt. Als Lösungsmittel für die Metallsalzprecursoren eignen sich Wasser genauso wie organische Lösungsmittel. Besonders geeignet sind Wasser und niedere Alkohole wie Methanol und Ethanol.

Geeignete Precursoren bei der Verwendung von Edelmetallen als dehydrieraktive Komponente sind auch die entsprechenden Edelmetallsole, die nach einem der bekannten Verfahren, zum Beispiel durch Reduktion eines Metallsalzes in Gegenwart eines Stabilisators wie PVP mit einem Reduktionsmittel hergestellt werden können. Die Herstelltechnik wird in der deutschen Patentanmeldung DE 195 00 366 ausführlich behandelt.

Der Gehalt der erfindungsgemäßen Dehydrierungskatalysatoren an einem Edelmetall als dehydrieraktiver Komponente beträgt 0 bis 5 Gew.%, bevorzugt 0.05 bis 1 Gew.-%, besonders bevorzugt 0.05 bis 0.5 Gew.%.

Die weiteren Komponenten der Aktivmasse können entweder während der Herstellung des Trägers, zum Beispiel durch gemeinsame Fällung, oder nachträglich, zum Beispiel durch Tränken des Trägers mit geeigneten Precursor-Verbindungen, aufgebracht werden. Als Precursor-Verbindungen verwendet man in der Regel Verbindungen, die sich durch Calcinieren in die entsprechenden Oxide umwandeln lassen. Geeignet sind zum Beispiel Hydroxide, Carbonate, Oxalate, Acetate, Chloride oder gemischte Hydroxycarbonate der entsprechenden Metalle.

In vorteilhaften Ausführungsformen enthält die Aktivmasse folgende weitere Komponenten:
- mindestens ein Element aus der I. oder II. Hauptgruppe, bevorzugt Caesium und/oder Kalium mit einem Gehalt zwischen 0 und 20 Gew.%, bevorzugt zwischen 0,1 und 15 Gew.%, besonders bevorzugt zwischen und 0,2 und 10 Gew.-%;
- mindestens ein Element der III. Nebengruppe einschließlich der Lanthaniden und Actiniden, bevorzugt Lanthan und/oder Cer mit einem Gehalt zwischen 0 und 20 Gew.-%, bevorzugt zwischen zwischen 0,1 und 15 Gew.-%, besonders bevorzugt zwischen und 0,2 und 10 Gew.-%;
- mindestens ein Element aus der III. und IV. Hauptgruppe, bevorzugt Zinn mit einem Gehalt zwischen 0 und 10 Gew.-%.

Der Dehydrierungskatalysator ist bevorzugt halogenfrei.

Der Dehydrierungskatalysator kann im Reaktor fest angeordnet oder z.B. in Form eines Wirbelbettes verwendet werden und eine entsprechende Gestalt haben. Geeignet sind z.B. Formen wie Splitt, Tabletten, Monolithe, Kugeln, oder Extrudate (Stränge, Wagenräder, Sterne, Ringe).

Als dehydrierbare Kohlenwasserstoffe können Paraffine, Alkylaromaten, Naphthene oder Olefine mit 2 bis 30 C-Atomen eingesetzt werden. Das Verfahren ist dabei besonders geeignet für die Dehydrierung von gerad- oder verzweigtkettigen Kohlenwasserstoffen mit einer Kettenlänge von 2 bis 15 Kohlenstoffatomen, bevorzugt mit 2 bis 5 Kohlenstoffatomen. Beispiele sind Ethan, Propan, n-Butan, iso-Butan, n-Pentan, isoPentan, n-Hexan, n-Heptan, n-Octan, n-Nonan, n-Decan, n-Undecan, n-Dodecan, n-Tridecan, n-Tetradecan, n-Pentadecan. Besonders bevorzugter Kohlenwasserstoff ist Propan. In der weiteren Beschreibung der Erfindung wird des öfteren auf diesen besonders bevorzugten Fall der Propan-Dehydrierung eingegangen, die entsprechenden Merkmale gelten jedoch in analoger Weise auch für andere dehydrierbare Kohlenwasserstoffe.

Da die Dehydrierreaktion unter Volumenzunahme verläuft, kann der Umsatz durch Erniedrigung des Partialdrucks der Reaktanten gesteigert werden. Dies lässt sich in einfacher Weise z.B. durch Dehydrierung bei vermindertem Druck und/oder durch Zumischen eines Inertgases erreichen. Geeignete Inertgase stellen z.B. Stickstoff, Wasserdampf, Kohlendioxid und Edelgase wie He, Ne oder Ar dar. Bevorzugt sind unter den Reaktionsbedingungen inerte (d.h. zu weniger als 5 mol%, bevorzugt zu weniger als 3 mol-% und noch besser zu weniger als 1 mol-% sich chemisch verändernde) Verdünnungsmittel. Eine Verdünnung mit Wasserdampf bedingt als weiteren Vorteil in der Regel ein vermindertes Verkoken des erfindungsgemäß eingesetzten Dehydrierungskatalysators und damit eine erhöhte Standzeit, da der Wasserdampf mit gebildetem Koks nach dem Prinzip der Kohlevergasung abreagiert. Das Verhältnis von Wasserdampf zu dem zu dehydrierenden Kohlenwasserstoff liegt dabei im Bereich zwischen 0 und 10 mol/mol, bevorzugt zwischen 0,1 und 5 mol/mol.

Das erfindungsgemäße Verfahren wird in mindestens einer Reaktionszone unter gleichzeitiger Wärmeerzeugung durch exotherme Reaktion von Wasserstoff, Kohlenwasserstoff und/oder Kohlenstoff in Gegenwart eines sauerstoffhaltigen Gases durchgeführt. Im allgemeinen beträgt die insgesamt zugeführte Sauerstoffmenge, bezogen auf die Gesamtmenge des zu dehydrierenden Kohlenwasserstoffes, 0,001 bis 0,5 mol/mol, bevorzugt 0,005 bis 0,2 mol/mol, besonders bevorzugt 0,05 bis 0,2 mol/mol. Im allgemeinen wird die Menge des dem Reaktionsgasgemisch zugesetzten sauerstoffhaltigen Gases so gewählt, dass durch die Verbrennung des im Reaktionsgasgemisch vorhandenen Wasserstoffs oder Kohlenwasserstoffs und/oder des in Form von Koks vorliegenden Kohlenstoffs die für die Dehydrierung des Kohlenwasserstoffs zum Alken benötigte Wärmemenge erzeugt wird. In besonderen Ausführungsformen kann die durch Verbrennung mit Sauerstoff erzeugte Wärme auch größer oder kleiner sein als die für die Dehydrierung des Kohlenwasserstoffs benötigte Wärme. Sauerstoff kann entweder als reiner Sauerstoff oder im Gemisch mit Inertgasen wie CO₂, N₂ oder den Edelgasen eingesetzt werden. Bevorzugtes sauerstoffhaltiges Gas ist Luft. Alternativ zu molekularem Sauerstoff können auch weiter sauerstoffhaltige gasförmige Oxidationsmittel, beispielsweise Distickstoffoxid oder Ozon, eingesetzt werden. Die Inertgase und die resultierenden Verbrennungsgase wirken im allgemeinen zusätzlich verdünnend und fordern damit die heterogen katalysierte Dehydrierung.

Der zur Wärmeerzeugung verbrannte Wasserstoff kann der bei der Dehydrierung gebildete Wasserstoff oder dem Reaktionsgasgemisch zusätzlich zugesetzter Wasserstoff sein.

In einer Ausführungsform der Erfindung wird dem Reaktionsgasgemisch kein Wasserstoff zugesetzt und die zur Dehydrierung benötigte Wärme zumindest teilweise durch Verbrennung (exotherme Reaktion) von Kohlenwasserstoff und des bei der Dehydrierung gebildeten Wasserstoffs erzeugt.

In einer weiteren Ausführungsform wird dem Reaktionsgasgemisch zusätzlich Wasserstoff zugesetzt.

Der erfindungsgemäß eingesetzte Dehydrierungskatalysator katalysiert im allgemeinen auch die Verbrennung der Kohlenwasserstoffe und von Wasserstoff mit Sauerstoff, so dass grundsätzlich kein von diesem verschiedener spezieller Oxidationskatalysator erforderlich ist. In einer Ausführungsform wird neben dem Dehydrierungskatalysator ein von diesem verschiedener spezieller Oxidationskatalysator eingesetzt, der selektiv die Oxidation von Wasserstoff zur Wärmeerzeugung katalysiert, insbesondere dann, wenn zusätzlicher Wasserstoff zugesetzt wird.

Wird, wie es einer Ausführungsform der Erfindung entspricht, dem Reaktionsgasgemisch kein zusätzlicher Wasserstoff zugesetzt, so kann die Dehydrierungswärme ohne weiteres durch katalytische Verbrennung der Kohlenwasserstoffe und von bei der Dehydrierung gebildetem Wasserstoff an dem Dehydrierungskatalysator erzeugt werden. Geeignete, sauerstoffunempfindliche Dehydrierungskatalysatoren, die die Verbrennung der Kohlenwasserstoffe katalysieren, sind die oben beschriebenen Lewis-sauren Katalysatoren. Bevorzugt sind die oben beschriebenen Dehydierungskatalysatoren, die mindestens ein Element der VIII. Nebengruppe, mindestens ein Element der I. und/oder II. Hauptgruppe, mindestens ein Element der III. und/oder IV. Hauptgruppe und mindestens ein Element der III. Nebengruppe einschließlich der Lanthaniden und Actiniden auf Zirkonoxid und/oder Siliziumdioxid als Träger enthalten.

In einer bevorzugten Ausführungsform wird zur direkten Wärmeerzeugung durch Verbrennung dem Reaktionsgasgemisch Wasserstoff zugesetzt. Im allgemeinen wird dem Reaktionsgasgemisch soviel Wasserstoff zugesetzt, dass das Molverhältnis H₂/O₂ im Reaktionsgasgemisch unmittelbar nach der Einspeisung 0,1 bis 200 mol/mol, vorzugsweise 1 bis 20 mol/mol, besonders bevorzugt 2 bis 10 mol/mol beträgt. Dies gilt bei mehrstufigen Reaktoren für jede Zwischeneinspeisung von Wasserstoff und Sauerstoff.

Die Wasserstoffverbrennung erfolgt katalytisch. In einer Ausführungsform der Erfindung wird kein von dem Dehydrierungskatalysator verschiedener spezieller Oxidationskatalysator eingesetzt. In einer besonders bevorzugten Ausführungsform wird in Gegenwart eines oder mehrerer Oxidationskatalysatoren gearbeitet, die selektiv die Verbrennung von Wasserstoff zu Sauerstoff in Gegenwart von Kohlenwasserstoffen katalysieren. Die Verbrennung der Kohlenwasserstoffe mit Sauerstoff zu CO und CO₂ läuft dadurch nur in untergeordnetem Maße ab, was sich deutlich positiv auf die erzielten Selektivitäten für die Bildung von Alkenen auswirkt. Vorzugsweise liegen der Dehydrierungskatalysator und der Oxidationskatalysator in verschiedenen Reaktionszonen vor.

Bei mehrstufiger Reaktionsführung kann der Oxidationskatalysator in nur einer, in mehreren oder in allen Reaktionszonen vorliegen.

Bevorzugt ist der Katalysator, der selektiv die Oxidation von Wasserstoff in Gegenwart von Kohlenwasserstoffen katalysiert, an den Stellen angeordnet, an denen höhere Sauerstoffpartialdrucke herrschen als an anderen Stellen des Reaktors, insbesondere in der Nähe der Einspeisungsstelle für das sauerstoffhaltige Gas. Die Einspeisung von sauerstofmaltigem Gas und/oder Wasserstoff kann an einer oder mehreren Stelle des Reaktors erfolgen.

Ein bevorzugter Katalysator, der selektiv die Verbrennung von Wasserstoff katalysiert, enthält Oxide oder Phosphate, ausgewählt aus der Gruppe bestehend aus den Oxiden oder Phosphaten von Germanium, Zinn, Blei, Arsen, Antimon oder Bismut.
Ein weiterer bevorzugter Katalysator, der die Verbrennung von Wasserstoff katalysiert, enthält ein Edelmetall der VIII. oder I. Nebengruppe.
Bei heterogen katalysierten Dehydrierungen von Kohlenwasserstoffen werden im allgemeinen mit der Zeit geringe Mengen an schwersiedenden, hochmolekularen organischen Verbindungen oder Kohlenstoff gebildet, die sich auf der Katalysatoroberfläche abscheiden und den Katalysator mit der Zeit deaktivieren. Die erfindungsgemäß eingesetzten Dehydrierungskatalysatoren zeichnet sich durch eine geringe Neigung zur Koksbildung und eine geringe Geschwindigkeit der Deaktivierung aus.

Mit den erfindungsgemäß eingesetzten Dehydrierungskatalysatoren lassen sich hohe Raum-Zeit-Ausbeuten erzielen, die für die Dehydrierung von Propan bei über 2 kg Propen / kg Katalysator ·· h liegen und damit deutlich über den Raum-Zeit-Ausbeuten der Verfahren nach dem Stand der Technik. Durch Verdünnung des Reaktionsgasgemisches mit Inertgas, durch Erhöhung der Reaktionstemperatur und/oder durch Absenken des Reaktionsdruckes lassen sich die thermodynamisch möglichen Grenzumsätze so weit erhöhen, dass sie deutlich über den angestrebten Reaktionsumsätzen liegen. Auf diese Weise lassen sich mit den erfindungsgemäß eingesetzten Katalysatoren Raum-Zeit-Ausbeuten von über 6 kg Propen / kg Katalysator · h erzielen. Die Belastung (GHSV) des Katalysators kann bei dieser auch als Hochlastfahrweise bezeichneten Fahrweise
> 8000 h⁻¹ betragen.

Zur Regeneration des Dehydrierungskatalysators können die an sich bekannten Verfahren eingesetzt werden. So kann, wie oben beschrieben, dem Reaktionsgasgemisch Wasserdampf zugesetzt werden. Sich abscheidender Kohlenstoff wird unter diesen Reaktionsbedingungen nach dem Prinzip der Kohlevergasung teilweise oder vollständig entfernt.

Alternativ dazu kann von Zeit zu Zeit ein Sauerstoff enthaltendes Gas bei erhöhter Temperatur über die Katalysatorschüttung geleitet werden und der abgeschiedene Kohlenstoff abgebrannt werden.

Nach längerer Betriebsdauer wird der erfindungsgemäß eingesetzte Dehydrierungskatalysator bevorzugt dadurch regeneriert, dass bei einer Temperatur von 300 bis 600°C, häufig bei 350 bis 500°C, zunächst einen Spülgang mit Inertgas durchführt wird und anschließend in einer ersten Regenerierstufe mit Stickstoff verdünnte Luft über das Katalysatorbett geleitet wird. Die Katalysatorbelastung beträgt dabei vorzugsweise 50 bis 10 000 h⁻¹ und der Sauerstoffgehalt ca. 0,5 bis 2 Vol.%. In weiteren sich anschließenden Regenerierstufen wird der Sauerstoffgehalt sukzessive auf ca. 20 Vol.-% (reine Luft) erhöht. Vorzugsweise werden 2 bis 10, besonders bevorzugt 2 bis 5 Regenerierstufen durchgeführt. Im allgemeinen wird anschließend noch mit reinem Wasserstoff oder mit einem Inertgas verdünntem Wasserstoff (Wasserstoffgehalt > 1 Vol-%) unter ansonsten gleichen Bedingungen regeneriert. Bevorzugt werden alle Regenerierstufen in Gegenwart von Wasserdampf durchgeführt.

Das erfindungsgemäße Verfahren kann grundsätzlich in allen aus dem Stand der Technik bekannten Reaktortypen und Fahrweisen durchgeführt werden. Die zusätzliche Einspeisung von Sauerstoff führt dazu, dass zumindest ein Teil der Reaktionswärme bzw. der Energie, die zum Aufheizen des Reaktionsgasgemisches erforderlich ist, durch die direkte Verbrennung aufgebracht wird und nicht indirekt über Wärmeaustauscher übertragen werden muss.

Eine ausführliche Beschreibung von geeigneten Reaktortypen und Fahrweisen enthält auch "Catalytica® Studies Division, Oxidative Deydrogenation and Alternative Deydrogenation Processes, Study Number 4192 OD, 1993, 430 Ferguson Drive, Mountain View, California, 94043-5272 U.S.A."

Eine geeignete Reaktorform ist der Festbettrohr- oder Rohrbündelreaktor. Bei diesen befindet sich der Katalysator (Dehydrierungs- und gegebenenfalls spezieller Oxidationskatalysator) als Festbett in einem Reaktionsrohr oder in einem Bündel von Reaktionsrohren. Die Reaktionsrohre werden üblicherweise dadurch indirekt beheizt, dass in dem die Reaktionsrohre umgebenden Raum ein Gas, z.B. ein Kohlenwasserstoff wie Methan, verbrannt wird. Günstig ist es dabei, diese indirekte Form der Aufheizung lediglich auf den ersten ca. 20 bis 30% der Länge der Festbettschüttung anzuwenden und die verbleibende Schüttungslänge durch die im Rahmen der indirekten Aufheizung freigesetzte Strahlungswärme auf die erforderliche Reaktionstemperatur aufzuheizen. Das indirekte Aufheizen des Reaktionsgases kann erfindungsgemäß in vorteilhafter Weise mit dem direkten Aufheizen durch Verbrennung in dem Reaktionsgasgemisch gekoppelt werden. Durch Kopplung der direkten Wärmeeinbringung mit der indirekten Wärmeinbringung ist eine annähernd isotherme Reaktionsführung erreichbar. Übliche Reaktionsrohr-Innendurchmesser betragen etwa 10 bis 15 cm. Ein typischer Dehydrierrohrbündelreaktor umfasst ca. 300 bis 1000 Reaktionsrohre. Die Temperatur im Reaktionsrohrinneren bewegt sich üblicherweise im Bereich von 300 bis 700°C, vorzugsweise im Bereich von 400 bis 700°C. Der Arbeitsdruck liegt üblicherweise zwischen 0,5 und 8 bar, häufig zwischen 1 und 2 bar bei Verwendung einer geringen Wasserdampfverdünnung (entsprechend dem BASF-Linde-Verfahren), aber auch zwischen 3 und 8 bar bei Verwendung einer hohen Wasserdampfverdünnung (entsprechend dem sogenannten "steam active reforming process" (STAR-Prozess) von Phillips Petroleum Co., siehe US 4,902,849, US 4,996,387 und US 5,389,342). In der Regel verlässt das Produktgemisch das Reaktionsrohr mit einer 50 bis 100°C tieferen Temperatur. Typische Katalysatorbelastungen mit Propan liegen bei 500 bis 2000 h⁻¹. Die Katalysatorgeometrie kann beispielsweise kugelförmig oder zylindrisch (hohl oder voll) sein.

Das erfindungsgemäße Verfahren kann in einem Wanderbett-Reaktor durchgeführt werden. Beispielsweise kann das Katalysator-Wanderbett in einem Radialstromreaktor untergebracht sein. In diesem bewegt sich der Katalysator langsam von oben nach unten, während das Reaktionsgasgemisch radial fließt. Diese Verfahrensweise wird beispielsweise im sogenannten UOP-Oleflex-Dehydrierverfahren angewandt. Da die Reaktoren bei diesem Verfahren quasi adiabat betrieben werden, ist es zweckmäßig, mehrere Reaktoren hintereinander geschaltet zu betreiben (in typischer Weise bis zu vier Reaktoren). Vor oder in jedem Reaktor wird in das Eintrittsgasgemisch durch Verbrennung in Gegenwart des zugeführten Sauerstoffs auf die erforderliche Reaktionstemperatur aufgeheizt. Durch die Verwendung mehrerer Reaktoren lassen sich große Unterschiede der Temperaturen des Reaktionsgasgemisches zwischen Reaktoreingang und Reaktorausgang vermeiden und trotzdem hohe Gesamtumsätze erzielen.

Wenn das Katalysatorbett den Wanderbettreaktor verlassen hat, wird es der Regenerierung zugeführt und anschließend wiederverwendet. Der erfindungsgemäß eingesetzte Dehydrierungskatalysator weist im allgemeinen Kugelform auf. Dem zu dehydrierenden Kohlenwasserstoff, vorzugsweise Propan, kann Wasserstoff zugegeben werden, auch um eine schnelle Katalysatordeaktivierung zu vermeiden. Der Arbeitsdruck liegt typischerweise bei 2 bis 5 bar. Das Molverhältnis von Wasserstoff zu Propan beträgt vorzugsweise von 0,1 bis 10. Die Reaktionstemperaturen liegen bevorzugt bei 550 bis 660°C.

Eine Kohlenwasserstoff-Dehydrierung nach dem erfindungsgemäßen Verfahrens kann auch, wie in Chem. Eng. Sci. 1992 b, 47 (9-11) 2313 beschrieben, heterogen katalysiert im Wirbelbett durchgeführt werden, wobei der Kohlenwasserstoff nicht verdünnt wird. Zweckmäßigerweise werden dabei zwei Wirbelbetten nebeneinander betrieben, von denen sich eines in der Regel im Zustand der Regenerierung befindet. Der Arbeitsdruck beträgt typischerweise 1 bis 2 bar, die Dehydriertemperatur in der Regel 550 bis 600°C. Die für die Dehydrierung erforderliche Wärme wird dabei in das Reaktionssystem eingebracht, indem der Dehydrierkatalysator auf die Reaktionstemperatur vorerhitzt wird. Durch erfindungsgemäße Verwendung eines sauerstoffhaltigen Co-Feeds kann auf die Vorerhitzer verzichtet werden, und die benötigte Wärme direkt im Reaktorsystem durch Verbrennung in Gegenwart von Sauerstoff erzeugt werden.

In einer besonders bevorzugten Ausführungsform des erfindungsgemäßen Verfahrens wird die Dehydrierung in einem Hordenreaktor durchgeführt. Dieser enthält ein oder mehrere aufeinanderfolgende Katalysatorbetten. Die Anzahl der Katalysatorbetten kann 1 bis 20, zweckmäßigerweise 2 bis 8, insbesondere 4 bis 6 betragen. Die Katalysatorbetten werden vorzugsweise radial oder axial vom Reaktionsgas durchströmt. Im allgemeinen wird ein solcher Hordenreaktor mit einem Katalysatorfestbett betrieben.

Im einfachsten Fall sind die Katalysatorfestbetten in einem Schachtofenreaktor axial oder in den Ringspalten von zentrisch ineinander gestellten zylindrischen Gitterrosten angeordnet. Ein Schachtofenreaktor entspricht einer Horde. Die Durchführung des erfindungsgemäßen Verfahrens in einem einzelnen Schachtofenreaktor ist möglich, aber weniger bevorzugt

Bei einer Fahrweise ohne Sauerstoff als Co-Feed wird das Reaktionsgasgemisch im Hordenreaktor auf seinem Weg von einem Katalysatorbett zum nächsten Katalysatorbett einer Zwischenerhitzung unterworfen, z.B. durch Überleiten über mit heißen Gasen erhitzte Wärmeaustauscherrippen oder durch Durchleiten durch mit heißen Brenngasen beheizte Rohre.

In dem erfindungsgemäßen Verfahren wird die vorstehend geschilderte Zwischenerhitzung zumindest teilweise auf direktem Weg durchgeführt. Dazu wird dem Reaktionsgasgemisch entweder bereits vor Durchströmen des ersten Katalysatorbetts und/oder zwischen den nachfolgenden Katalysatorbetten in begrenztem Umfang molekularer Sauerstoff zugesetzt.

An dem erfindungsgemäß eingesetzten Katalysator werden so in begrenztem Umfang im Reaktionsgasgemisch enthaltene Kohlenwasserstoffe, bereits auf der Katalysatoroberfläche abgeschiedener Koks oder kohleähnliche Verbindungen, aber auch im Verlauf der Dehydrierung gebildeter Wasserstoff verbrannt. Die dabei freigesetzte Reaktionswärme ermöglicht so eine nahezu isotherme Betriebsweise der heterogen katalysierten Kohlenwasserstoffdehydrierung. Dabei kann mit oder ohne zusätzliche Einspeisung von Wasserstoff gearbeitet werden.

In einer Ausführungsform der Erfindung erfolgt eine Zwischeneinspeisung von sauerstoffhaltigem Gas und gegebenenfalls Wasserstoff vor jeder Horde des Hordenreaktors. In einer weiteren Ausführungsform des erfindungsgemäßen Verfahrens erfolgt die Einspeisung von sauerstoffhaltigem Gas und gegebenenfalls Wasserstoff vor jeder Horde außer der ersten Horde. In einer bevorzugten Ausführungsform wird unter Zwischeneinspeisung von Wasserstoff gearbeitet, wobei in einer speziellen Ausführungsform hinter jeder Einspeisungsstelle eine Schicht aus einem speziellen Oxidationskatalysator vorhanden ist, gefolgt von einer Schicht aus dem Dehydrierungskatalysator, und in einer zweiten speziellen Ausführungsform kein spezieller Oxidationskatalysator vorhanden ist. In einer weiteren bevorzugten Ausführungsform wird ohne Einspeisung von Wasserstoff gearbeitet.

Die Dehydriertemperatur beträgt im allgemeinen 400 bis 800 °C, der Druck im allgemeinen 0,2 bis 5 bar, bevorzugt 0,5 bis 2 bar, besonders bevorzugt 1 bis 1,5 bar. Die Belastung (GHSV) beträgt im allgemeinen 500 bis 2000 h⁻¹, bei Hochlastfahrweise auch bis zu 16000 h⁻¹, bevorzugt 4000 bis 16000 h⁻¹.

Der in dem erfindungsgemäßen Verfahren eingesetzte Kohlenwasserstoff muss kein Reinstoff sein. Vielmehr kann der eingesetzte Kohlenwasserstoff andere dehydrierbare Gase wie Methan, Ethan, Ethylen, Propan, Propen, Butane, Butene, Propin, Acetylen, H₂S oder Pentane enthalten. Insbesondere kann die erfindungsgemäße Dehydrierung auch mit großtechnisch erzeugten und in großen Mengen zur Verfügung stehenden Alkan-Gemischen wie LPG (liquified petroleum gas) durchgeführt werden. Auch können aus einem anderen Verfahren herrührende Kreisgase, beispielsweise wie in der deutschen Patentanmeldung P 10028582.1 beschrieben, verwendet werden.

Die Aufarbeitung des Reaktoraustrags erfolgt in an sich bekannter Weise, beispielsweise durch Abtrennung des im Produktgemisch enthaltenen molekularen Wasserstoffs, Abtrennung der von Alkanen und Alkenen verschiedenen Bestandteile, bevorzugt durch selektive Absorption des Alken/Alkan-Gemischs in einem organischen Lösungsmittel und Auftrennung des Alken/Alkan-Gemischs in einem C₃-Splitter und Rückführung des Alkans in die Dehydrierung.

Die Erfindung wird durch die nachstehenden Beispiele näher erläutert.

### Beispiele

### Beispiel 1

1000 g eines gesplitteten ZrO₂·SiO₂-Mischoxides der Firma Norton (Siebfraktion 1,6 bis 2 mm) wurden mit einer Lösung von 11,992 g SnCl₂·2H₂O und 7,888 g H₂PtCl₆·6H₂O in 5950 ml Ethanol übergossen.

Das überstehende Ethanol wurde am Rotationsverdampfer abgezogen. Anschließend wurde 15 h bei 100°C getrocknet und 3 h bei 560°C calciniert. Danach wurde der erhaltene Katalysator mit einer Lösung von 7,68 g CsNO₃, 13,54 g KNO₃ und 98,329 g La(NO₃)₃•6H₂O in 23 ml H₂O übergossen. Das überstehende Wasser wurde am Rotationsverdampfer abgezogen. Anschließend wurde 15 h bei 100°C getrocknet und 3 h bei 560°C calciniert.

Der Katalysator hatte eine BET-Oberfläche von 85 m²/g. Quecksilber-Porosimetrie-Messungen ergaben ein Porenvolumen von 0,29 ml/g.

### Beispiel 2

55 g eines gesplitteten ZrO₂•SiO₂ Mischoxides der Firma Norton (Siebfraktion 1,6 bis 2 mm) wurden mit einer Lösung von 0,6 g SnCl₂•2H₂O und 0,394 g H₂PtCl₆•6H₂O in 300 ml Ethanol übergossen.

Das überstehende Ethanol wurde am Rotationsverdampfer abgezogen. Anschließend wurde 15 h bei 100°C getrocknet und 3 h bei 560°C calciniert. Danach wurde der Katalysator mit einer Lösung von 0,386 g CsNO₃, 0,680 g KNO₃ und 4,888 g Ce(NO₃)₃•6H₂O in 130 ml H₂O übergossen. Das überstehende Wasser wurde am Rotationsverdampfer abgezogen. Anschließend wurde 15 h bei 100°C getrocknet und 3 h bei 560°C calciniert.

Der Katalysator hatte eine BET-Oberfläche von 72,4 m²/g. Quecksilber-Porosimetrie-Messungen ergaben ein Porenvolumen von 0,26 ml/g.

### Beispiel 3

57 g eines gesplitteten ZrO₂ Trägers der Firma Norton (Siebfraktion 1,6 bis 2 mm) wurden mit einer Lösung von 0,684 g SnCl₂·2H₂O und 0,45 g H₂PtCl₆·6H₂O in 342 ml Ethanol übergossen.

Das überstehende Ethanol wurde am Rotationsverdampfer abgezogen. Anschließend wurde 15 h bei 100°C getrocknet und 3 h bei 560°C calciniert. Danach wurde der Katalysator mit einer Lösung von 0,44 g CsNO₃, 0,775 g KNO₃ und 5,604 g Ce(NO₃)₃•6H₂O in 148 ml H₂O übergossen. Das überstehende Wasser wurde am Rotationsverdampfer abgezogen. Anschließend wurde 15 h bei 100°C getrocknet und 3 h bei 560°C calciniert.

Der Katalysator hatte eine BET-Oberfläche von 40 m²/g. Quecksilber-Porosimetrie-Messungen ergaben ein Porenvolumen von 0,25 ml/g.

### Beispiel 4

In einem 5L-Rührkolben wurden 521,3 g Zr(OH)₄ in 2000 ml H₂O suspendiert. Zur Suspension wurden 73,53 g SiO₂-Ludox-Sol (47,6 Gew.-% SiO₂-Gehalt) gegeben. Die Suspension wurde 4 h bei Raumtemperatur gerührt. Anschließend wurde das Produkt sprühgetrocknet. Die Kopftemperatur wurde auf 350°C eingestellt, die Ausgangstemperatur lag bei 105 bis 110°C, der Sprühdruck betrug 2,5 bar. Die Sprühscheibe rotierte mit einer Geschwindigkeit von 28 000 U/min. Das resultierende weiße Pulver zeigte einen Glühverlust von 15,1 %.
471,15 g des weißen Pulvers wurde zusammen mit 133,30g Pural SCF (Al₂O₃) und 30,22g konzentrierter HNO₃ 2 h geknetet. Mittels einer Strangpresse (Pressdruck 75bar) wurde die Paste zu 3 mm-Strängen geformt. Die Stränge wurden 4 h bei 200°C getrocknet und 2 h bei 600°C calciniert. Anschließend wurden die Stränge zu Partikeln einer Siebfraktion von 1,6 bis 2 mm gesplittet.
60 g des so hergestellten Trägers wurden mit einer Lösung von 0,712 g SnCl₂·2H₂O und 0,468 g H₂PtCl₆·6H₂O in 368 ml Ethanol übergossen.

Das überstehende Ethanol wurde am Rotationsverdampfer abgezogen. Anschließend wurde 15 h bei 100°C getrocknet und 3 h bei 560°C calciniert. Danach wurde der Katalysator mit einer Lösung von 0,458 g CsNO₃, 0,807 g KNO₃ und 5,838 g La(NO₃)₃•6H₂O in 157 ml H₂O übergossen. Das überstehende Wasser wurde am Rotationsverdampfer abgezogen. Anschließend wurde 15 h bei 100°C getrocknet und 3 h bei 560°C calciniert.

Der Katalysator hatte eine BET-Oberfläche von 98 m²/g. Quecksilber-Porosimetrie-Messungen ergaben ein Porenvolumen von 0,35 ml/g.

### Beispiel 5

23 g eines gesplitteten Mg(Al)O-Trägers der Firma Giulini (Siebfraktion 1,6 bis 2 mm) wurden bei 700°C 2h calciniert. Anschließend wurde der Träger mit einer Lösung von 0,276 g SnCl₂•2H₂O und 0,181 g H₂PtCl₆•6H₂O in 138 ml Ethanol übergossen.

Das überstehende Ethanol wurde am Rotationsverdampfer abgezogen. Anschließend wurde 15 h bei 100°C getrocknet und 3 h bei 560°C calciniert. Danach wurde der Katalysator mit einer Lösung von 0,177 g CsNO₃, 0,313 g KNO₃ und 2,262 g La(NO₃)₃•6H₂O in 60 ml H₂O übergossen. Das überstehende Wasser wurde am Rotationsverdampfer abgezogen. Anschließend wurde 15 h bei 100°C getrocknet und 3 h bei 560°C calciniert.

Der Katalysator hatte eine BET-Oberfläche von 103 m²/g. Quecksilber-Porosimetrie-Messungen ergaben ein Porenvolumen von 0,51 ml/g.

### Beispiel 6

57 g eines gesplitteten theta-Al₂O₃-Trägers der Firma Condea (Siebfraktion 1,6 bis 2 mm) wurden mit einer Lösung von 0,3718 g SnCl₂·2H₂O und 0,245 g H₂PtCl₆·6H₂O in 190 ml Ethanol übergossen.

Das überstehende Ethanol wurde am Rotationsverdampfer abgezogen. Anschließend wurde 15 h bei 100°C getrocknet und 3 h bei 560°C calciniert. Danach wurde der Katalysator mit einer Lösung von 0,239 g CsNO₃, 0,4214 g KNO₃ und 5,604 g La(NO₃)₃•6H₂O in 80 ml H₂O übergossen. Das überstehende Wasser wurde am Rotationsverdampfer abgezogen. Anschließend wurde 15 h bei 100°C getrocknet und 3 h bei 560°C calciniert.

Der Katalysator hatte eine BET-Oberfläche von 119 m²/g. Quecksilber-Porosimetrie-Messungen ergaben ein Porenvolumen von 0,66 ml/g.

### Beispiel 7

23 g eines gesplitteten theta-Al₂O₃-Trägers der Firma BASF (Siebfraktion 1,6 - 2 mm) wurden mit einer Lösung von 0,2758 g SnCl₂•2H₂O und 0,1814 g H₂PtCl₆•6H₂O in 138 ml Ethanol übergossen.

Das überstehende Ethanol wurde am Rotationsverdampfer abgezogen. Anschließend wurde 15 h bei 100°C getrocknet und 3 h bei 560°C calciniert. Danach wurde der Katalysator mit einer Lösung von 0,1773 g CsNO₃ , 0,3127 g KNO₃ und 2,26 g La(NO₃)₃ 6H₂O in 60 ml H₂O übergossen. Das überstehende Wasser wurde am Rotationsverdampfer abgezogen. Anschließend wurde 15 h bei 100°C getrocknet und 3 h bei 560°C calciniert.

Der Katalysator hatte eine BET-Oberfläche von 34 m²/g. Quecksilber-Porosimetrie-Messungen ergaben ein Porenvolumen von 0,23 ml/g.

### Beispiel 8

43,25g (NH₄)₂CO₃ wurden in 1 1 Wasser gelöst und mit 849 ml einer 25 gew.-%igen ammoniakalischen Lösung vermischt und auf 75°C erwärmt. In die Lösung wurden 2333,3g Mg(NO₃)₂•6H₂O und 337,6g Al(NO₃)₃•9H₂O, gelöst in 3 1 Wasser, über einen Tropftrichter unter Rühren schnell zugegeben. Nachdem 1 Stunde bei 75°C nachgerührt worden war, wurde der entstandene Niederschlag abfiltriert, der Filterkuchen mit Wasser gewaschen. Anschließend wurde 15 h bei 100°C getrocknet und 2 h bei 900°C calciniert.

Das Pulver wurde mit 3 Gew.-% Magnesiumstearat vermischt und auf einer Exzenterpresse zu 20 x 2 mm-Tabletten vorkompaktiert.

33 g des so hergestellten Mg(Al)O-Trägers wurden gesplittet (Siebfraktion 1,6 bis 2 mm) und bei 700°C für 2 Stunden calciniert. Anschließend wurde der Träger mit einer Lösung von 0,398 g SnCl₂•2H₂O und 0,262 g H₂PtCl₆•6H₂O in 200 ml Ethanol übergossen.

Das überstehende Ethanol wurde am Rotationsverdampfer abgezogen. Anschließend wurde 15 h bei 100°C getrocknet und 3 h bei 560°C calciniert. Danach wurde der Katalysator mit einer Lösung von 0,256 g CsNO₃ , 0,451 g KNO₃ und 3,265 g La(NO₃)₃•6H₂O in 87 ml H₂O übergossen. Das überstehende Wasser wurde am Rotationsverdampfer abgezogen. Anschließend wurde 15 h bei 100°C getrocknet und 3 h bei 560°C calciniert.

Der Katalysator hatte eine BET-Oberfläche von 85 m²/g. Quecksilber-Porosimetrie-Messungen ergaben ein Porenvolumen von 0.28 ml/g.

### Beispiel 9

### Katalysatortest:

20 ml des zuvor hergestellten Katalysators wurden in einen Rohrreaktor mit einem Innendurchmesser von 20 mm eingebaut. Der Katalysator wurde 30 min bei 500°C mit Wasserstoff versetzt. Danach wurde der Katalysator bei der gleichen Temperatur einem Gemisch aus 80 Vol.-% Stickstoff und 20 Vol.-% Luft (Magerluft) ausgesetzt. Nach einer Spülphase von 15 min mit reinem Stickstoff wurde der Katalysator 30 min mit Wasserstoff reduziert. Danach wurde der Katalysator bei einer Reaktionstemperatur von 610°C mit 20 Nl/h Propan (99.5 Vol.-%) und H₂O im Molverhältnis Propan/Wasserdampf von 1 : 1 beaufschlagt. Der Druck betrug 1,5 bar, die Belastung (GHSV) betrug 2000 h⁻¹. Die Reaktionsprodukte wurden gaschromatographisch analysiert. Die Ergebnisse sind in der Tabelle zusammengefasst.

Die Brönsted- bzw. Lewis-Acidität der in den Beispielen 1 bis 8 hergestellten Katalysatoren wurden mit dem Sondengas Pyridin unter Verwendung einer HV-FTIR-Meßzelle charakterisiert.

Die Proben wurden 1 Stunde bei 390°C unter Luftatmosphäre aufgeheizt, anschließend auf 10⁻⁵ mbar evakuiert, auf 80°C abgekühlt und mit Pyridin bei 3 mbar Gleichgewichtsdruck begast. Zur Prüfung der Evakuierungsstabilität des Pyridinadsorbates wurde die bei 3 mbar begaste Probe einer Vakuumbehandlung bei Ölpumpenvakuum (ca. 10⁻² mbar, 3min) und bei Hochvakuum (ca. 10⁻⁵ mbar, 1 h) unterzogen. Dabei wurden Physisorbate desorbiert. Die Adsorbatspektren wurden im Hochvakuum aufgenommen.

Die gemessenen Extinktionen wurden auf die Schichtdicke bezogen (in integralen Extinktionseinheiten (IEE) pro µm Schichtdicke). Als Hintergrund der Adsorbatspektren diente das Single-beam-Spektrum der unbegasten, auf 80°C abgekühlten Probe unter Hochvakuum. Matrixbanden wurden dabei vollständig wegkompensiert.

Ausgewertet wurden die Bande bei 1440 cm⁻¹ (entspricht Lewis-sauren Zentren) und zusätzlich die Kontrollbande bei 1490 cm⁻¹ (entspricht Lewis-saueren Zentren, falls keine Brönsted-sauren Zentren vorhanden).

Die Ergebnisse sind in der Tabelle zusammengefasst.

Alle untersuchten Proben zeigten keine messbare Brönsted-Acidität. Die gemessene Lewis-Acidität korreliert gut mit den Umsätzen der Propandehydrierung.

**Tabelle**

| Beispiel | Träger | Lewis-Ac (AU). | Propan-Umsatz (%) |
|---|---|---|---|
| 1 | ZrO₂/SiO₂ (Norton) | 8,97 | 46,8 |
| 2 | ZrO₂/SiO₂ (Ce statt La) | 7,82 | 47,2 |
| 3 | ZrO₂ (Norton) | 4,59 | 30 |
| 4 | ZrO₂/SiO₂/Al₂O₃ | 7,29 | 42,1 |
| 5 | Mg(Al)O (Giulini) | 2,88 | 12,4 |
| 6 | theta-Al₂O₃ (Condea) | 3,51 | 30,4 |
| 7 | theta-Al₂O₃ (BASF) | 1,77 | 11,4 |
| 8 | Mg(Al)O | 1,66 | 12,2 |

Alle Träger sind mit Pt_{0.3}/Sn_{0.6}/Cs_{0.5}/K_{0.5}/La_{3.0} beladen.

### Beispiel 10

### Hochlastfahrweise

2,5 ml des gemäß Beispiel 1 hergestellten Katalysators wurden mit 77,5 .ml Steatit verdünnt und in einen Rohrreaktor mit 20 mm Innendurchmesser eingebaut. Der Katalysator wurde nacheinander für je 30 Minuten bei 500°C zunächst mit Wasserstoff, dann mit Magerluft (80 Vol.% Stickstoff und 20 Vol.% Luft) und anschließend erneut mit Wasserstoff versetzt. Die Vorgänge wurden stets durch ein 15 minütiges Spülen mit Stickstoff zeitlich voneinander getrennt. Anschließend wurde der Katalysator bei 600°C mit 20 NL/h Propan (99,5 Vol.-%) und Wasserdampf im Molverhältnis Propan/H₂O von 1:1 beaufschlagt. Der Druck betrug 1,5 bar, die Belastung (GHSV) betrug 16 000 h⁻¹. Die Reaktionsprodukte wurden gaschromatographisch analysiert. Nach einer Stunde Reaktionszeit wurden 30% des eingesetzten Propans mit einer Selektivität zu Propen von 95% umgesetzt. Die Raum-Zeit-Ausbeute an Propen bezogen auf das eingesetzte Katalysatorvolumen betrug 8 g Propen / (g Katalysator * h).

### Beispiel 11

### Sauerstofffahrweise

20 ml des gemäß Beispiel 1 hergestellten Katalysators wurden in einen Rohrreaktor mit 20 mm Innendurchmesser eingebaut. Der Katalysator wurde nacheinander für je 30 Minuten bei 500°C zunächst mit Wasserstoff, dann mit Magerluft (80 Vol.-% Stickstoff und 20 Vol.-% Luft) und anschließend erneut mit Wasserstoff versetzt. Die Vorgänge wurden stets durch 15-minütiges Spülen mit Stickstoff zeitlich voneinander getrennt. Anschließend wurde der Katalysator bei 610°C mit 20 NL/h Propan (99,5 Vol.-%) und Wasserdampf im Molverhältnis Propan/H₂O von 1:1 beaufschlagt. Zusätzlich wurde Sauerstoff im Molverhältnis Propan/O₂ von 20:1 zugeführt. Der Druck betrug 1,5 bar, die Belastung (GHSV) betrug 2100 h⁻¹. Die Reaktionsprodukte wurden gaschromatographisch analysiert. Nach einer Stunde Reaktionszeit wurden 50% des eingesetzten Propans mit einer Selektivität zu Propen von 90% umgesetzt. Nach einer Reaktionszeit von 16 h lag der Umsatz bei 44% und die Selektivität bei 90%.

### Beispiel 12

### Sauerstofffahrweise bei kleinem Umsatz

20 ml des gemäß Beispiel 1 hergestellten Katalysators wurden in einen Rohrreaktor mit 20 mm Innendurchmesser eingebaut. Der Katalysator wurde nacheinander für je 30 Minuten bei 500°C zunächst mit Wasserstoff, dann mit Magerluft (80 Vol.-% Stickstoff und 20 Vol.% Luft) und anschließend erneut mit Wasserstoff versetzt. Die Vorgänge wurden stets durch 15-minütiges Spülen mit Stickstoff zeitlich voneinander getrennt. Anschließend wurde der Katalysator bei 500°C mit 20 NL/h Propan (99,5 Vol.-%) und Wasserdampf im Molverhältnis Propan/H₂O von 1 : 1 beaufschlagt. Zusätzlich wurde Sauerstoff im Molverhältnis Propan/O₂ von 20 : 1 zugeführt. Der Druck betrug 1,5 bar, die Belastung (GHSV) betrug 2100 h⁻¹. Die Reaktionsprodukte wurden gaschromatographisch analysiert. Nach einer Stunde Reaktionszeit wurden 16% des eingesetzten Propans mit einer Selektivität zu Propen von 99% umgesetzt. Nach einer Reaktionszeit von 100 h lag der Umsatz bei 14% und die Selektivität bei 94%. Nach einer Temperaturerhöhung auf 510°C lag der Propanumsatz nach 300 h bei 15% und die Selektivität bei 94%. Nach einer weiteren Temperaturerhöhung auf 530°C lag wurden nach 800 h 15% des eingesetzten Propans mit einer Selektivität zu Propen von 94% umgesetzt. Die Propanzufuhr und die Wasserzufuhr wurden nach 1700 h abgestellt und Magerluft (80 Vol.-% Stickstoff und 20 Vol.% Luft) bei 400°C über den Katalysator geleitet. Anschließend wurde für 30 min reine Luft über den Katalysator geleitet. Nachdem der Reaktor 15 min mit Stickstoff gespült wurde, wurde für 30 min Wasserstoff über den Katalysator geleitet. Nachdem erneut Propan, Wasserdampf und Sauerstoff als Feed zugegeben wurden, konnten bei 505°C 15% Propanumsatz bei 92% Selektivität erzielt werden. Nach insgesamt 2300 h lag der Propanumsatz bei 540°C bei 15% und die Selektivität zu Propen bei 94%.

### Beispiel 13

### Sauerstofffahrweise bei kleinem Umsatz mit zusätzlicher N₂-Verdünnung

Der Katalysator aus Beispiel 1 wurde nach 2300 h erneut (nach Abstellen von Propan- und Wasserdampfzufuhr) mit Magerluft (80 Vol.-% Stickstoff und 20 Vol.-% Luft) bei 400°C beaufschlagt. Anschließend wurde für 30 min reine Luft über den Katalysator geleitet. Nachdem der Reaktor 15 min mit Stickstoff gespült worden war, wurde für 30 min Wasserstoff über den Katalysator geleitet. Anschließend wurde Propan, Stickstoff, Sauerstoff und Wasserdampf im Verhältnis 5,8/7,8/0,4/5,8 bei 505°C über den Katalysator geleitet. Der Reaktionsdruck betrug 1,5 bar, die Belastung (GHSV) betrug 1300 h⁻¹. Der Propanumsatz lag bei 20% bei einer Selektivität von 92%. Nach 500 h wurden bei 540°C 20% Propan mit einer Selektivität zu Propen von 92% umgesetzt.

### Beispiel 14

### Sauerstofffahrweise bei kleinem Umsatz mit zusätzlicher N₂-Verdünnung und zusätzlicher H₂-Zugabe

Im Rahmen der Versuchsdurchführung aus Beispiel 12 wurde nach einer Laufzeit von insgesamt 2500 h zusätzlich Wasserstoff dem Feed beigemengt. Die Feed setzte sich demnach wie folgt zusammen: C3/N₂/O₂/H₂/H₂O wie 5,8/7,8/0,4/0,8/5,8. Der Reaktionsdruck betrug 1,5 bar, die Belastung (GHSV) betrug 1300 h⁻¹. Die Reaktionstemperatur wurde auf 575°C eingestellt. Der Propanumsatz lag bei 20% bei einer Propen-Selektivität von 92%. Der zugeführte Sauerstoff wurde vollständig umgesetzt. 60% des zugeführten Sauerstoffs reagierten dabei mit Propan bzw. Propen zu Kohlendioxid und Kohlenmonoxid, 40% des zugeführten Sauerstoffs reagierten mit zugeführtem oder durch die Dehydrierung gebildetem Wasserstoff zu Wasser.

### Beispiel 15

### Regenerierung des Katalysators

1000 ml des gemäß Beispiel 6 hergestellten Katalysators wurden mit 500 ml Steatit verdünnt und in einen Rohrreaktor mit 40 mm Innendurchmesser eingebaut Der Katalysator wurde nacheinander für je 30 Minuten bei 500°C zunächst mit Wasserstoff, dann mit Magerluft (80 Vol.-% Stickstoff und 20 Vol.-% Luft) und anschließend erneut mit Wasserstoff versetzt. Die Vorgänge wurden stets durch ein 15-minütiges Spülen mit Stickstoff zeitlich voneinander getrennt. Anschließend wurde der Katalysator bei 610°C mit 250 NL/h Propan (99,5 Vol.-%) und Wasserdampf im Molverhältnis Propan/H₂O von 1:1 beaufschlagt. Der Druck betrug 1,5 bar, die Belastung (GHSV) betrug 500 h⁻¹. Die Reaktionsprodukte wurden gaschromatographisch analysiert. Nach einer Stunde Reaktionszeit wurden 55% des eingesetzten Propans mit einer Selektivität zu Propen von 90% umgesetzt. Nach einer Reaktionszeit von 12 h lag der Umsatz bei 53% und die Selektivität bei 93%. Die Propanzufuhr und die Wasserzufuhr wurden und Magerluft (92 Vol.-% Stickstoff und 8 Vol.-% Luft) bei 400°C über den Katalysator geleitet. Anschließend wurde der Luftgehalt zweimalig erhöht (zunächst 83 Vol.-% Stickstoff und 17 Vol.-% Luft, dann 64 Vol.-% Stickstoff und 36 Vol.-% Luft). Anschließend wurde reine Luft über den Katalysator geleitet bis der CO₂-Austrag kleiner 0,04 Vol-% betrug. Nachdem der Reaktor 15 Minuten mit Stickstoff gespült worden war, wurde für 30 min Wasserstoff über den Katalysator geleitet. Nachdem erneut Propan, Wasserdampf und Sauerstoff als Feed zugegeben wurden, konnten bei 610°C 55% Propanumsatz bei 92% Selektivität erzielt werden. Nach 10-maligem Regenerieren des Katalysators in der vorbeschriebenen Weise konnten bei 610°C 54% Umsatz bei einer Propen-Selektivität von 93% erzielt werden. Nach 30-maligem Regenerieren konnten bei 610°C 54% Umsatz bei 93% Propen-Selektivität erzielt werden.

## Patentansprüche

1. Verfahren zur heterogen katalysierten Dehydrierung in einer oder mehreren Reaktionszonen von einem oder mehreren dehydrierbaren C₂-C₃₀- Kohlenwasserstoffen in einem diese enthaltenden Reaktionsgasgemisch, wobei zumindest ein Teil der benötigten Dehydrierwärme in mindestens einer Reaktionszone durch Verbrennung von Wasserstoff, des oder der Kohlenwasserstoffe und/oder von Kohlenstoff in Gegenwart eines sauerstoffhaltigen Gases direkt in dem Reaktionsgasgemisch erzeugt wird, **dadurch gekennzeichnet, dass** das Reaktionsgasgemisch, welches den oder die dehydrierbaren Kohlenwasserstoffe enthält, mit einem Lewis-aciden Dehydrierungskatalysator in Kontakt gebracht wird, der eine Brönsted-Acidität kleiner 0,1 Aciditätseinheiten (AU) aufweist, wobei der Dehydrierungskatalysator eine Lewis-Acidität größer 3 Aciditätseinheiten (AU), bestimmbar aus IR-Absorptionspektren von an dem Katalysator adsorbiertem Pyridin, aufweist, wobei 1 AU dem tausendfachen der gemessenen Extinktion (angegeben in integralen Extinktionseinheiten IEE) geteilt durch die Schichtdicke (in µm), die sich bei der Bestimmung der Lewis- und Brönsted-Acidität der Dehydrierungskatalysatoren mit dem Sondengas Pyridin ergibt, entspricht.

2. Verfahren nach Anspruch 1, **dadurch gekennzeichnet, dass** der Dehydrierungskatalysator ein Metalloxid, ausgewählt aus der Gruppe bestehend aus Zirkondioxid, Zinkoxid, Aluminiumoxid, Siliciumdioxid, Titandioxid, Magnesiumoxid, Lanthanoxid und Ceroxid, enthält.

3. Verfahren nach Anspruch 2, **dadurch gekennzeichnet, dass** der Dehydrierungskatalysator Zirkondioxid und/oder Siliziumdioxid enthält.

4. Verfahren nach Anspruch 1 bis 3 **dadurch gekennzeichnet, dass** der Dehydrierungskatalysator mindestens ein Element der VIII. Nebengruppe, mindestens ein Element der I. oder II. Hauptguppe, mindestens ein Element der III. oder IV. Hauptgruppe und mindestens ein Element der III. Nebengruppe, einschließlich der Lanthaniden und Actiniden, enthält.

5. Verfahren nach einem der Ansprüche 1 bis 4, **dadurch gekennzeichnet, dass** der Dehydrierungskatalysator Platin und/oder Palladium enthält.

6. Verfahren nach einem der Ansprüche 1 bis 5, **dadurch gekennzeichnet, dass** der Dehydrierungskatalysator Caesium und/oder Kalium enthält.

7. Verfahren nach einem der Ansprüche 1 bis 6, **dadurch gekennzeichnet, dass** der Dehydrierungskatalysator Lanthan und/oder Cer enthält.

8. Verfahren nach einem der Ansprüche 1 bis 7, **dadurch gekennzeichnet, dass** der Dehydrierungskatalysator Zinn enthält.

9. Verfahren nach einem der Ansprüche 1 bis 8, **dadurch gekennzeichnet, dass** der Dehydrierungskatalysator eine bimodale Porenradienverteilung aufweist, wobei 70% bis 100% der Poren einen Porendurchmesser kleiner als 20 nm oder zwischen 40 und 5000 nm aufweisen.

10. Verfahren nach einem der Ansprüche 1 bis 9, **dadurch gekennzeichnet, dass** das Reaktionsgasgemisch Wasserdampf enthält.

11. Verfahren nach einem der Ansprüche 1 bis 10, **dadurch gekennzeichnet, dass** dem Reaktionsgasgemisch Wasserstoff zugesetzt wird.

12. Verfahren nach Anspruch 11, **dadurch gekennzeichnet, dass** wenigstens eine Reaktionszone einen Katalysator enthält, der selektiv die Verbrennung von Wasserstoff mit Sauerstoff in Gegenwart von Kohlenwasserstoffen katalysiert.

13. Verfahren nach einem der Ansprüche 1 bis 12, **dadurch gekennzeichnet, dass** der Katalysator, der die Verbrennung von Wasserstoff katalysiert, Oxide oder Phosphate, ausgewählt aus der Gruppe bestehend aus den Oxiden oder Phosphaten von Germanium, Zinn, Blei, Arsen, Antimon oder Bismut, enthält.

14. Verfahren nach einem der Ansprüche 1 bis 13, **dadurch gekennzeichnet, dass** der Katalysator, der selektiv die Verbrennung von Wasserstoff katalysiert, ein Edelmetall der VIII. oder I. Nebengruppe enthält.

15. Verfahren nach einem der Ansprüche 1 bis 14, **dadurch gekennzeichnet, dass** die Dehydrierung in einem Hordenreaktor durchgeführt wird.

## Claims

1. A process for the heterogeneously catalyzed dehydrogenation in one or more reaction zones of one or more dehydrogenatable C₂-C₃₀-hydrocarbons in a reaction gas mixture comprising them, with at least part of the heat of dehydrogenation required being generated directly in the reaction gas mixture in at least one reaction zone by combustion of hydrogen, the hydrocarbon or hydrocarbons and/or carbon in the presence of an oxygen-containing gas, wherein the reaction gas mixture comprising the dehydrogenatable hydrocarbon or hydrocarbons is brought into contact with a Lewis-acid dehydrogenation catalyst which has a Brönsted acidity of less than 0.1 acidity units (AU), where the dehydrogenation catalyst has a Lewis acidity of greater than 3 acidity units (AU), determinable from IR absorption spectra of pyridine adsorbed on the catalyst, where 1 AU corresponds to one thousand times the measured absorbance (reported in integrated extinction units IEE) divided by the thickness of the sample (in µm) obtained in the determination of the Lewis and Brönsted acidities of the dehydrogenation catalysts using the probe gas pyridine.

2. The process according to claim 1, wherein the dehydrogenation catalyst comprises a metal oxide selected from the group consisting of zirconium dioxide, zinc oxide, aluminum oxide, silicon dioxide, titanium dioxide, magnesium oxide, lanthanum oxide and cerium oxide.

3. The process according to claim 2, wherein the dehydrogenation catalyst comprises zirconium dioxide and/or silicon dioxide.

4. The process according to any of claims 1 to 3, wherein the dehydrogenation catalyst comprises at least one element of transition group VIII, at least one element of main group I or II, at least one element of main group III or IV and at least one element of transition group III including the lanthanides and actinides.

5. The process according to any of claims 1 to 4, wherein the dehydrogenation catalyst comprises platinum and/or palladium.

6. The process according to any of claims 1 to 5, wherein the dehydrogenation catalyst comprises cesium and/or potassium.

7. The process according to any of claims 1 to 6, wherein the dehydrogenation catalyst comprises lanthanum and/or cerium.

8. The process according to any of claims 1 to 7, wherein the dehydrogenation catalyst comprises tin.

9. The process according to any of claims 1 to 8, wherein the dehydrogenation catalyst has a bimodal pore radius distribution in which from 70% to 100% of the pores have a pore diameter less than 20 nm or in the range from 40 to 5000 nm.

10. The process according to any of claims 1 to 9, wherein the reaction gas mixture comprises water vapor.

11. The process according to any of claims 1 to 10, wherein hydrogen is added to the reaction gas mixture.

12. The process according to claim 11, wherein at least one reaction zone contains a catalyst which selectively catalyzes the combustion reaction of hydrogen and oxygen in the presence of hydrocarbons.

13. The process according to any of claims 1 to 12, wherein the catalyst which catalyzes the combustion of hydrogen comprises oxides or phosphates selected from the group consisting of the oxides and phosphates of germanium, tin, lead, arsenic, antimony and bismuth.

14. The process according to any of claims 1 to 13, wherein the catalyst which selectively catalyzes the combustion of hydrogen comprises a noble metal of transition group VIII or I.

15. The process according to any of claims 1 to 14, wherein the dehydrogenation is carried out in a tray reactor.

## Revendications

1. Procédé de déshydrogénation à catalyse hétérogène, dans une ou plusieurs zones réactionnelles, d'un ou de plusieurs hydrocarbures en C₂-C₃₀ déshydrogénables dans un mélange gazeux réactionnel contenant ceux-ci, dans lequel au moins une partie de la chaleur de déshydrogénation nécessaire est produite directement dans le mélange gazeux réactionnel, dans au moins une zone réactionnelle, par combustion d'hydrogène, du ou des hydrocarbures et/ou de carbone en présence d'un gaz contenant de l'oxygène, **caractérisé en ce que** le mélange gazeux réactionnel, qui contient le ou les hydrocarbures déshydrogénables, est mis en contact avec un catalyseur de déshydrogénation acide de Lewis qui présente une acidité de Brönsted inférieure à 0,1 unité d'acidité (AU), le catalyseur de déshydrogénation présentant une acidité de Lewis supérieure à 3 unités d'acidité (AU), déterminable à partir de spectres d'absorption IR de pyridine adsorbée sur le catalyseur, 1 AU correspondant à mille fois l'extinction mesurée (indiquée en unités d'extinction intégrales IEE) divisée par l'épaisseur de couche (en *µ*m) qu'on obtient lors de la détermination de l'acidité de Lewis et de Brônsted des catalyseurs de déshydrogénation avec le gaz échantillon, la pyridine.

2. Procédé suivant la revendication 1, **caractérisé en ce que** le catalyseur de déshydrogénation contient un oxyde métallique, choisi parmi le groupe constitué du dioxyde de zirconium, de l'oxyde de zinc, de l'oxyde d'aluminium, du dioxyde de silicium, du dioxyde de titane, de l'oxyde de magnésium, de l'oxyde de lanthane et de l'oxyde de cérium.

3. Procédé suivant la revendication 2, **caractérisé en ce que** le catalyseur de déshydrogénation contient du dioxyde de zirconium et/ou du dioxyde de silicium.

4. Procédé suivant les revendications 1 à 3, **caractérisé en ce que** le catalyseur de déshydrogénation contient au moins un élément du groupe secondaire VIII, au moins un élément du groupe principal I ou II, au moins un élément du groupe principal III ou IV et au moins un élément du groupe secondaire III, y compris les lanthanides et les actinides.

5. Procédé suivant l'une des revendications 1 à 4, **caractérisé en ce que** le catalyseur de déshydrogénation contient du platine et/ou du palladium.

6. Procédé suivant l'une des revendications 1 à 5, **caractérisé en ce que** le catalyseur de déshydrogénation contient du césium et/ou du potassium.

7. Procédé suivant l'une des revendications 1 à 6, **caractérisé en ce que** le catalyseur de déshydrogénation contient du lanthane et/ou du cérium.

8. Procédé suivant l'une des revendications 1 à 7, **caractérisé en ce que** le catalyseur de déshydrogénation contient de l'étain.

9. Procédé suivant l'une des revendications 1 à 8, **caractérisé en ce que** le catalyseur de déshydrogénation présente une répartition bimodale des rayons des pores, 70 % à 100 % des pores présentant un diamètre de pore inférieur à 20 nm ou entre 40 et 5 000 nm.

10. Procédé suivant l'une des revendications 1 à 9, **caractérisé en ce que** le mélange gazeux réactionnel contient de la vapeur d'eau.

11. Procédé suivant l'une des revendications 1 à 10, **caractérisé en ce que** de l'hydrogène est ajouté au mélange gazeux réactionnel.

12. Procédé suivant la revendication 11, **caractérisé en ce qu'**au moins une zone réactionnelle contient un catalyseur qui catalyse de manière sélective la combustion d'hydrogène avec de l'oxygène en présence d'hydrocarbures.

13. Procédé suivant l'une des revendications 1 à 12, **caractérisé en ce que** le catalyseur, qui catalyse la combustion d'hydrogène, contient des oxydes ou des phosphates choisis parmi le groupe constitué des oxydes ou phosphates de germanium, d'étain, de plomb, d'arsenic, d'antimoine ou de bismuth.

14. Procédé suivant l'une des revendications 1 à 13, **caractérisé en ce que** le catalyseur, qui catalyse de manière sélective la combustion d'hydrogène, contient un métal noble du groupe secondaire VIII ou I.

15. Procédé suivant l'une des revendications 1 à 14, **caractérisé en ce que** la déshydrogénation est effectuée dans un réacteur à claies.
